# EUROPÄISCHE PATENTANMELDUNG

(11) **EP 1 797 957 A1**
(43) Veröffentlichungstag der Anmeldung: **20.06.2007**
(21) Anmeldenummer: 05027546.0
(22) Anmeldetag: 15.12.2005
(51) Int. Cl.: B01L 3/02, A61M 5/28

(54) **Pipettiervorrichtung**

(71) Anmelder: Dentaco Dentalindustrie- und Marketing GmbH, 61352 Bad Homburg (DE)
(72) Erfinder: Sogaro, Alberto C., 61476 Kronberg (DE)
(74) Vertreter: Jung Schirdewahn Grünberg Schneider

(57) **Zusammenfassung**

Es wird eine Pipettiervorrichtung (10) vorgeschlagen, die eine elastisch verformbare röhrchenartige Pipettiereinheit (12), die einen Aufnahmeraum (26) aufweist und ein erstes, mit einer Ausgabeöffnung (14) ausgebildetes Ende und ein zweites vorzugsweise offenes Ende hat. Im Bereich des zweiten Endes ist ein kapselartiger Einsatz (20) fixiert, der in den Aufnahmeraum (26) eintaucht und öffenbar ausgebildet ist, so dass bei einem unter Verformung der Pipettiereinheit (12) erfolgenden Öffnen des Einsatzes (20) eine fließfähige Substanz aus mindestens einem Vorratsraum (28) des Einsatzes (20) in den Aufnahmeraum (26) der Pipettier-einheit (12) strömt und durch lateralen Druck auf die Pipettiereinheit (12) appliziert werden kann.

## Beschreibung

Die Erfindung betrifft eine Pipettiervorrichtung, umfassend eine röhrchenartige Pipettiereinheit, die einen Aufnahmeraum hat und ein erstes, mit einer Ausgabeöffnung ausgebildetes Ende und ein zweites, der Ausgabeöffnung abgewandtes Ende aufweist.

Derartige Pipettiervorrichtungen sind aus der Praxis bekannt und beispielsweise mit einer aus Kunststoff oder Glas gefertigten Pipettiereinheit versehen. An einem Ende der Pipettiereinheit ist eine Saugeinrichtung aufgesetzt, so dass über die Ausgabe- bzw. Austrittsöffnung eine zu applizierende Flüssigkeit in den Aufnahmeraum gezogen und wieder auf ein Substrat oder in ein Reaktionsgefäß appliziert werden kann. Die zu applizierende Flüssigkeit kann insbesondere zu chemischen bzw. biochemischen Zwecken eingesetzt werden.

Bei einer aus Kunststoff gefertigten Pipettiereinheit ist es nicht möglich, aggressive Substanzen länger vorzuhalten, da diese den Kunststoff zerstören würden. Aus diesem Grunde werden derartige aggressive Substanzen bisher zumeist in Glasbehältern vorgehalten und dann nach einem Öffnen des Glasbehälters mittels einer Pipettiervorrichtung der einleitend genannten Art appliziert.

Der Erfindung liegt die Aufgabe zugrunde, eine Pipettiervorrichtung bereitzustellen, mittels der ein Applizieren einer Substanz in vereinfachter Weise erfolgen kann.

Diese Aufgabe ist erfindungsgemäß durch die Pipettiervorrichtung mit den Merkmalen des Patentanspruchs 1 gelöst.

Die Pipettiervorrichtung nach der Erfindung umfasst mithin eine elastisch verformbare, röhrchenartige Pipettiereinheit, die einen Aufnahmeraum aufweist und ein erstes, mit einer Ausgabeöffnung ausgebildetes Ende und ein zweites offenes Ende hat. Im Bereich des zweiten Endes ist ein kapselartiger Einsatz fixiert, der in den Aufnahmeraum eintaucht und aus einem zerbrechlichen Werkstoff gebildet ist, so dass bei einem Brechen des Einsatzes eine fließfähige Substanz aus mindestens einem Vorratsraum des Einsatzes in den Aufnahmeraum der Pipettiereinheit strömt und durch insbesondere lateralen Druck auf die Pipettiereinheit über die Ausgabeöffnung appliziert werden kann.

Der Kern der Erfindung besteht mithin darin, dass eine Pipettiervorrichtung selbst mit einer Kammer zum geschützten Vorhalten einer fließfähigen Substanz versehen ist, die erst unmittelbar vor dem eigentlichen Pipettiervorgang in einen Aufnahmeraum der Pipettiereinheit eingebracht werden kann. Dies erfolgt durch Zerstören des kapselartigen Einsatzes, was durch Ausüben eines radialen Drucks auf die elastisch verformbare Pipettiereinheit in Höhe des kapselartigen Einsatzes erfolgen kann.

In der Pipettiervorrichtung nach der Erfindung können auch aggressive Flüssigkeiten, wie Azeton, stabil gelagert werden, da der kapselartige Einsatz aus einem chemisch resistenten Werkstoff, wie Glas oder einem zerbrechlichen Kunststoff mit hoher chemischer Resistenz, gefertigt sein kann. Vor dem Applizieren bzw. Pipettieren ist kein Aufziehen der Pipettiervorrichtung mit der fließfähigen Substanz erforderlich. Die Aktivierung der Vorrichtung nach der Erfindung erfolgt allein durch Zerdrücken bzw. Brechen des Einsatzes, in dem die fließfähige Substanz bevorratet ist.

Bei einer bevorzugten Ausführungsform der Pipettiervorrichtung nach der Erfindung umfasst der kapselartige Einsatz einen plattenartigen Fixierabschnitt, der in Presssitz mit der Pipettiereinheit verbunden sein kann, und einen röhrchenartigen Vorratsabschnitt, in dem der Vorratsraum ausgebildet ist und der von der Wandung der Pipettiereinheit beabstandet ist. Bei dieser Ausführungsform ist gewährleistet, dass der Einsatz bzw. dessen Vorratsabschnitt den Aufnahmeraum der Pipettiereinheit nicht komplett ausfüllt, so dass auch nach der Zerstörung insbesondere des Vorratsabschnitts eine Dosierung zum Applizieren der fließfähigen Substanz problemlos durch lateralen Druck auf die Pipettiereinheit möglich ist.

Eine definierte Zerstörung des Einsatzes kann insbesondere dann erfolgen, wenn der Einsatz mit einer Sollbruchstelle versehen ist.

Die Sollbruchstelle kann in konstruktiv einfacher Weise durch eine Nut am Umfang des Einsatzes realisiert sein.

Bei einer speziellen Ausführungsform der Pipettiervorrichtung nach der Erfindung weist der Einsatz mindestens zwei Vorratsräume auf. In diesem Fall werden bei der Zerstörung des Einsatzes die in den einzelnen Vorratsräumen vorgehaltenen Substanzen in dem Aufnahmeraum der Pipettiereinheit vermischt. Eine derartig ausgebildete Pipettiervorrichtung ist also auch für Mehrstoffsysteme geeignet.

Denkbar ist es aber auch, eine Komponente eines Mehrstoffsystems in dem Aufnahmeraum der Pipettiereinheit und die andere(n) Komponente(n) des Mehrstoffsystems in dem Einsatz vorzuhalten, wobei eine Mischung der Komponenten erst beim Zerstören des Einsatzes erfolgt.

Besonders anwendungsfreundlich ist die Pipettiervorrichtung nach der Erfindung ausgelegt, wenn eine Zerbrechhilfe für den Einsatz bereitgestellt wird. Diese kann beispielsweise eine an der Innenwandung der Pipettiereinheit ausgebildete Einrichtung aus einem Hartstoff wie beispielsweise eine Rampe, ein Hebel oder dergleichen sein und zur Übertragung des manuell auf die Pipettiereinheit ausgeübten Drucks auf den Einsatz dienen.

Um zu verhindern, dass eine in dem Aufnahmeraum der Pipettiereinheit enthaltene Substanz in ungewünschter Weise über die Ausgabeöffnung austritt, kann die Ausgabeöffnung mit einem Verschluss versehen sein. Der Verschluss ist beispielsweise einstückig mit der Pipettiereinheit gefertigt und vor dem Einsatz der Pipettiervorrichtung abdrehbar, abziehbar oder abbrechbar. Denkbar ist es aber auch, den Verschluss als separates Bauteil in Form eines Stopfens oder einer Kappe auszubilden.

Des Weiteren kann die Pipettiervorrichtung nach der Erfindung im Bereich der Austrittsöffnung eine Applikationseinrichtung aufweisen, die die Form einer insbesondere biegsamen Pipettierspitze haben kann und auch mit einem pinselartigen oder auch flockenartigen Abschnitt versehen sein kann. Die Pipettierspitze kann wiederum einstückig mit der Pipettiereinheit gefertigt sein.

Weitere Vorteile und vorteilhafte Ausgestaltungen des Gegenstands nach der Erfindung sind der Beschreibung, der Zeichnung und den Patentansprüchen entnehmbar.

Ein Ausführungsbeispiel der Pipettiervorrichtung nach der Erfindung ist in der Zeichnung schematisch vereinfacht dargestellt und wird in der nachfolgenden Beschreibung näher erläutert. Es zeigt
- Fig .1: einen Längsschnitt durch eine erfindungsgemäß ausgelegte Pipettiervorrichtung; und

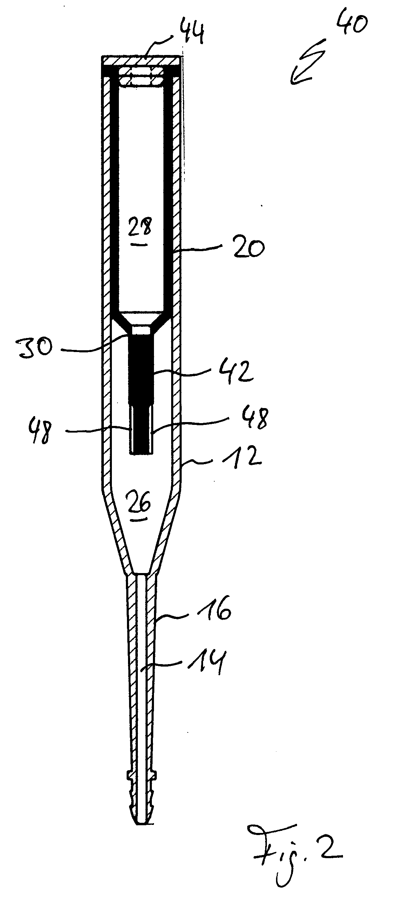

- Fig. 2: einen Längsschnitt durch eine alternative Ausführungsform einer erfindungsgemäß ausgelegten Pipettiervorrichtung.

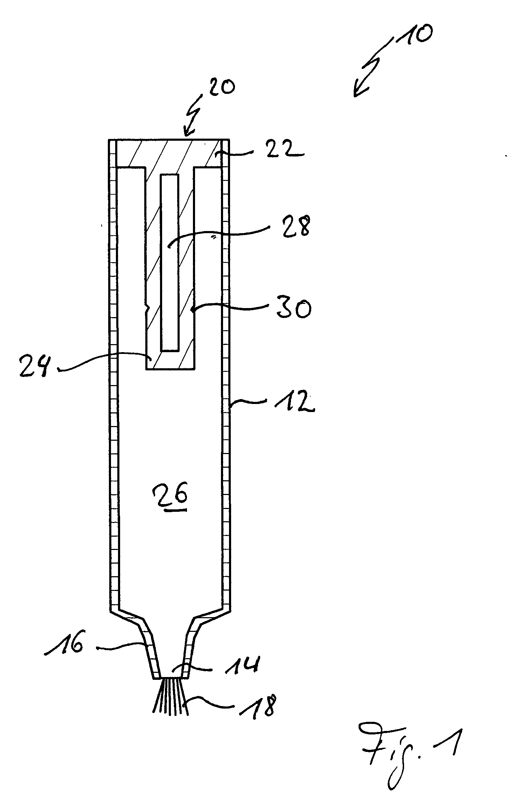

In Fig. 1 ist eine erfindungsgemäß ausgebildete Pipettiervorrichtung 10 dargestellt, die zum Applizieren einer aggressiven Flüssigkeit, wie Azeton, im Bereich der chemischen bzw. biochemischen Analytik eingesetzt werden kann.

Die Pipettiervorrichtung 10 umfasst eine röhrchenartige Pipettiereinheit 12, die aus einem elastisch verformbaren Kunststoff gefertigt ist und sich in Richtung einer Ausgabeöffnung 14 zu einer kapillarenartigen Pipettierspitze 16 verjüngt, die einstückig mit der Pipettiereinheit 12 gefertigt ist und an ihrem freien Ende mit einem Pinselhaar 18 versehen ist.

An ihrem der Ausgabeöffnung 14 abgewandten Ende ist die Pipettiereinheit 12 offen ausgebildet. Im Bereich dieses Endes ist in die Pipettiereinheit 12 ein kapselartiger Einsatz 20 eingeführt, der aus einem zerbrechlichen Werkstoff wie Glas gefertigt ist und zur Bevorratung der aggressiven Substanz dient. Der Einsatz 20 hat einen plattenartigen Fixierabschnitt 22, der einen kreisförmigen Grundriss hat und mit einem Durchmesser versehen ist, der im Wesentlichen mit dem Innendurchmesser der Pipettiereinheit 12 im Bereich von deren der Ausgabeöffnung 14 abgewandten Ende korrespondiert. An dem plattenartigen Fixierabschnitt 22 schließt sich ein röhrchenartiger Vorratsabschnitt 24 an, der in einen Aufnahmeraum 26 der Pipettiereinheit 12 derartig eintaucht, dass er frei hängend ist und mithin keinen Kontakt mit den Wänden der Pipettiereinheit 12 hat.

In dem Vorratsabschnitt 24 ist ein Vorratsraum 28 für die fließfähige Substanz ausgebildet. Der röhrenchenartige Vorratsabschnitt 24 ist an der dem plattenartigen Fixierabschnitt 22 abgewandten Ende verschlossen.

Des Weiteren hat der Vorratsabschnitt 24 an seinem Umfang eine Ringnut 30, die eine Sollbruchstelle des Einsatzes 20 bildet.

Die Anwendung der in Fig. 1 dargestellten Pipettiervorrichtung erfolgt in der Art, dass der Einsatz durch einen Anwender durch die elastisch verformbare Pipettiereinheit 12 hindurch im Bereich der von der Nut 30 gebildeten Sollbruchstelle durchbrochen und somit zerstört wird. Dadurch fließt die in dem Vorratsraum 28 enthaltene Flüssigkeit in den Aufnahmeraum 26 der Pipettiereinheit 12, so dass die Flüssigkeit durch einen seitlichen Druck auf die Pipettiereinheit 12 über die Pipettierspitze 16 aus der Ausgabeöffnung 14 zu dem Pinselhaar 18 ausgetragen und appliziert werden kann.

In Fig. 2 ist eine alternative Ausführungsform einer erfindungsgemäß ausgebildeten Pipettiervorrichtung 40 dargestellt, die zum Applizieren einer aggressiven Flüssigkeit, wie Azeton, im Bereich der chemischen bzw. biochemischen Analytik eingesetzt werden kann.

Die Pipettiervorrichtung 40 umfasst ebenfalls eine röhrchenartige Pipettiereinheit 12, die aus einem elastisch verformbaren Kunststoff gefertigt ist und sich in Richtung einer Ausgabeöffnung 14 zu einer kapillarenartigen Pipettierspitze 16 verjüngt, die einstückig mit der Pipettiereinheit 12 gefertigt ist und an ihrem freien Ende eine Pipettierspitze bildet.

An ihrem der Ausgabeöffnung 14 abgewandten Ende ist die Pipettiereinheit 12 offen ausgebildet. Im Bereich dieses Endes ist in die Pipettiereinheit 12 ein kapselartiger Einsatz 20 eingeführt, der aus einem zerbrechlichen Werkstoff wie Glas oder COC gefertigt ist und zur Bevorratung der aggressiven Substanz dient. Der Einsatz 20 hat ein offenes Befüllende mit einem Ringbund 46 und einen Durchmesser, der im Wesentlichen mit dem Innendurchmesser der Pipettiereinheit 12 im Bereich von deren der Ausgabeöffnung 14 abgewandten Ende korrespondiert und so im Presssitz mit der Pipettiereinheit verbunden ist. Das offene Befüllende ist mittels eines Deckels 44 verschlossen.

An der dem Deckel 44 abgewandten Ende ist an dem Einsatz 20 ein abbrechbarer Verschlusszapfen 42 angeformt, der unter Verformung der Pipettiereinheit 12 entlang einer Sollbruchstelle 30 abbrechbar ist, so dass eine in einem Vorratsraum 28 des Einsatzes 20 enthaltene Substanz in den Aufnahmeraum 26 der Pipettiereinheit strömen kann.

Am dem Vorratsraum 28 abgewandten Ende hat der Verschlusszapfen 42 am Umfang sich in axialer Richtung erstreckende Nuten 48, die gewährleisten, dass die Ausgabeöffnung 14 niemals komplett durch den Verschlusszapfen 42 verschlossen werden kann.

Die Anwendung der in Fig. 2 dargestellten Pipettiervorrichtung erfolgt in der Art, dass der Einsatz 20 durch einen Anwender durch die elastisch verformbare Pipettiereinheit 12 hindurch im Bereich der Sollbruchstelle 30 durchbrochen und somit zerstört wird. Dadurch fließt die in dem Vorratsraum 28 enthaltene Flüssigkeit in den Aufnahmeraum 26 der Pipettiereinheit 12, so dass die Flüssigkeit durch einen seitlichen Druck auf die Pipettiereinheit 12 über die Pipettierspitze 16 aus der Ausgabeöffnung 14 ausgetragen und appliziert werden kann.

## Patentansprüche

1. Pipettiervorrichtung, umfassend eine elastisch verformbare, röhrchenartige Pipettiereinheit (12), die einen Aufnahmeraum (26) aufweist und ein erstes, mit einer Ausgabeöffnung (14) ausgebildetes Ende und ein zweites, der Ausgabeöffnung (14) abgewandtes offenes Ende hat, **dadurch gekennzeichnet, dass** im Bereich des zweiten Endes ein kapselartiger Einsatz (20) fixiert ist, der in den Aufnahmeraum (26) eintaucht und derart öffenbar ausgebildet ist, dass bei einem unter Verformung der Pipettiereinheit (12) erfolgenden Öffnen des Einsatzes (20) eine fließfähige Substanz aus mindestens einem Vorratsraum (28) des Einsatzes (20) in den Aufnahmeraum (26) der Pipettiereinheit (12) strömt und durch lateralen Druck auf die Pipettiereinheit (12) appliziert werden kann.

2. Pipettiervorrichtung nach Anspruch 1, **dadurch gekennzeichnet, dass** der kapselartige Einsatz (20) aus einem zerbrechlichen Werkstoff gefertigt ist.

3. Pipettiervorrichtung nach Anspruch 1 oder 2, **dadurch gekennzeichnet, dass** der Einsatz (20) einen plattenartigen Fixierabschnitt (22), der vorzugsweise in Presssitz mit der Pipettiereinheit (12) verbunden ist, und einen röhrchenartigen Vorratsabschnitt (24) umfasst, der von der Wandung der Pipettiereinheit (12) beabstandet ist.

4. Pipettiervorrichtung nach einem der Ansprüche 1 bis 3, **dadurch gekennzeichnet, dass** der Einsatz (20') einen Deckel (44) aufweist, der im Presssitz fixiert ist.

5. Pipettiervorrichtung nach einem der Ansprüche 1 bis 3, **dadurch gekennzeichnet, dass** der Einsatz (20') einen abbrechbaren Verschlusszapfen (42) aufweist.

6. Pipettiervorrichtung nach Anspruch 1, **dadurch gekennzeichnet, dass** der Verschlusszapfen (42) mit axialen Nuten (48) versehen ist, die bei abgebrochenem Verschlusszapfen (42) einen Fluidstrom zu der Ausgabeöffnung (14) gewährleisten.

7. Pipettiervorrichtung nach einem der Ansprüche 1 bis 6, **dadurch gekennzeichnet, dass** der Einsatz (20) mit einer Sollbruchstelle (30) versehen ist.

8. Pipettiervorrichtung nach Anspruch 7, **dadurch gekennzeichnet, dass** die Sollbruchstelle als Nut (30) am Umfang des Einsatzes (20) gebildet ist.

9. Pipettiervorrichtung nach einem der Ansprüche 1 bis 8, **dadurch gekennzeichnet, dass** der Einsatz mindestens zwei Vorratsräume aufweist.

10. Pipettiervorrichtung nach einem der Ansprüche 1 bis 9, **gekennzeichnet durch** eine Zerbrechhilfe für den Einsatz.

11. Pipettiervorrichtung nach einem der Ansprüche 1 bis 10, **dadurch gekennzeichnet, dass** die Austrittsöffnung mit einem Verschluss versehen ist.

12. Pipettiervorrichtung nach einem der Ansprüche 1 bis 11, **dadurch gekennzeichnet, dass** im Bereich der Austrittsöffnung (14) eine Applikationseinrichtung angeordnet ist.

13. Pipettiervorrichtung nach Anspruch 12, **dadurch gekennzeichnet, dass** die Applikationseinrichtung eine Pipettierspitze (16) ist, die vorzugsweise einstückig mit der Pipettiereinheit (12) gefertigt ist.

14. Pipettiervorrichtung nach Anspruch 12 oder 13, **dadurch gekennzeichnet, dass** die Applikationseinrichtung mit einem pinselartigen oder flockenartigen Abschnitt (18) versehen ist.
